(19) Europäisches Patentamt
European Patent Office
Office européen des brevets

(11) EP 3 067 700 B1

(12) **EUROPEAN PATENT SPECIFICATION**

(45) Date of publication and mention of the grant of the patent:
**05.01.2022 Bulletin 2022/01**

(51) Int Cl.:
***G01N 33/94*** *(2006.01)* ***C07K 16/18*** *(2006.01)*

(21) Application number: **16160006.9**

(22) Date of filing: **11.03.2016**

(54) **IMMUNOASSAY FOR PREGABALIN**

IMMUNOASSAY FÜR PREGABALIN

DOSAGE IMMUNOLOGIQUE POUR LA PRÉGABALINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.03.2015 GB 201504174**

(43) Date of publication of application:
**14.09.2016 Bulletin 2016/37**

(73) Proprietor: **Randox Laboratories Ltd.**
**Crumlin, County Antrim BT29 4QY (GB)**

(72) Inventors:
- **Benchikh, Elouard**
**Crumlin, Antrim BT29 4QY (GB)**
- **McConnell, Ivan**
**Crumlin, Antrim BT29 4QY (GB)**
- **Fitzgerald, Peter**
**Crumlin, Antrim BT29 4QY (GB)**
- **Lowry, Philip**
**Crumlin, Antrim BT29 4QY (GB)**

(56) References cited:
**CN-U- 202 710 552** **TW-A- 201 040 523**
**US-A1- 2008 199 887**

- **KRASOWSKI MATTHEW D ET AL: "Advances in anti-epileptic drug testing", CLINICA CHIMICA ACTA, ELSEVIER BV, AMSTERDAM, NL, vol. 436, 10 June 2014 (2014-06-10), pages 224-236, XP029016171, ISSN: 0009-8981, DOI: 10.1016/J.CCA.2014.06.002**
- **JUENKE JOETTA M ET AL: "Performance characteristics of the ARK diagnostics gabapentin immunoassay", THERAPEUTIC DRUG MONITORING, LIPPINCOTT WILLIAMS AND WILKINS, NEW YORK, NY, US, vol. 33, no. 4, 1 August 2011 (2011-08-01) , pages 398-401, XP009188777, ISSN: 0163-4356, DOI: 10.1097/FTD.0B013E318220BB65**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 067 700 B1

## Description

### BACKGROUND OF THE INVENTION

One of the most common types of medications for which clinical laboratories carry out therapeutic drug monitoring (TDM) are anti-epileptic drugs (AEDs). Clinicians can utilize TDM of AEDs for a variety of reasons. For a drug which has a narrow therapeutic range it is important to monitor levels to avoid under or overdosing. Patients exhibit significant inter-individual variability in their pharmacokinetic responses to most AEDs so even in cases where a drug has a wide therapeutic range TDM can be used to monitor the clinical level which is most effective for an individual patient. In patients with pre-existing conditions such as renal failure it may be necessary to adjust the dosage to a lower but still effective level or to avoid gradual accumulation of the drug. TDM can also be used to assess adherence to therapy.

The anticonvulsant drug Pregabalin (3-(aminomethyl)-5-methylhexanoic acid) was designed as a more potent version of its predecessor gabapentin and, under the brand name Lyrical® it is one of the top selling drugs in the US. Pregabalin is not bound to plasma proteins and undergoes negligible metabolism with about 98% of a dose excreted unchanged in the urine. The only significant metabolite found is N-methyl pregabalin.

Methods currently available for the detection of pregabalin in human samples include High-performance liquid chromatography (HPLC) (*e.g.* Ahmadkhaniha *et al.* 2014), Liquid chromatography-tandem mass spectrometry (LC/MS/MS) (*e.g.* Mandal *et al.* 2008 and Krasowski et al. 2014 which teaches both HPLC and LC/MS/MS methods) and spectrofluorimetric methods (*e.g.* Shaalan, 2010). While these methods can be sensitive and reliable the equipment needed is expensive and so they are unavailable in most clinical laboratories. On the other hand, immunoassays are cost effective, simplistic and rapid alternatives to MS based analysis. Patent application US2008199887 teaches an immunoassay to gabapentin however, currently there are no available immunoassays for pregabalin TDM.

### References

Ahmadkhaniha, R et al. Chromatography Research International, vol. 2014, Article ID 450461, 6 pages, 2014. doi:10.1155/2014/450461.

Krasowski. M.D et al. Clinica Chimica Acta, 2014, vol. 436, pages 224-236. DOI: 10.1016/J.CCA.2014.06.002.

Mandal, U et al. Chromatographia, 2008, 67 (3-4); 237-243.

Shaalan, R. A-A, International Journal of Biomedical Science, 2010, 6(3); 260-267.


### Drawings

Figure 1, Structures of Pregabalin, 1,3-dimethylamylamine (DMAA), and Gabapentin.
Figure 2, Structures of Haptens A, B, C and D.
Figure 3, Chemical Reactions for the Synthesis of Pregabalin-aminoethylmaleimide (Hapten A).
Figure 4, Chemical Reactions for the Synthesis of Pregabalin-acetylthioprapanamine (Hapten B).
Figure 5, Chemical Reactions for the Synthesis of Pregabalin N-acetylthiopropioamide (Hapten C)
Figure 6, Chemical Reactions for the Synthesis of Pregabalin N-maleimidobutyramide (Hapten D)

### SUMMARY OF THE INVENTION

The current invention is defined in the appended claims. It provides the first known immunoassay for pregabalin. The immunoassay is underpinned by novel immunogens which are used to generate the novel antibodies of the assay. The invention will enable low cost and efficient TDM of this antiepileptic drug.

### DETAILED DESCRIPTION OF THE INVENTION

Unless otherwise stated technical terms as used herein are used according to the conventional usage as known to those skilled in the art.

The immunogen of the current invention is hapten A of Figure 2 coupled to an accm as defined in the claims.

The term "hapten" as used herein describes a pre-immunogenic molecule that stimulates antibody production only when conjugated to a larger carrier molecule. This larger carrier molecule can be referred to as an antigenicity-conferring carrier material (accm). Once the hapten is conjugated to the accm it forms the immunogen. In the immunogen

of the current invention the accm is coupled to the maleimide moiety of the hapten A. The hapten of the current invention may be either enantiomer of pregabalin or a racemic mixture of the two. Preferably the hapten is based on the (S) enantiomer of pregabalin. Preferably the starting materials for the examples given below also correspond to the (S) enantiomer however the (R) enantiomer or a mixture of the two may also be used as the starting material for the example reactions.

The term "immunogen" as used herein, describes an entity that induces an immune response such as production of antibodies or a T-cell response in a host animal.

The accm is selected from bovine serum albumin (BSA) and keyhole limpet haemocyanin (KLH).

Unless otherwise indicated, structures depicted herein are also meant to include all isomeric (e.g., enantiomeric, diastereomeric, cis-trans, conformational, and rotational) forms of the structure. For example, the R and S configurations for each asymmetric centre, (Z) and (E) double bond isomers, and (Z) and (E) conformational isomers are included in this invention, unless only one of the isomers is drawn specifically. As would be understood to one skilled in the art, a substituent can freely rotate around any rotatable bonds. Therefore, single stereochemical isomers as well as enantiomeric, diastereomeric, cis/trans, conformational, and rotational mixtures of the present compounds are within the scope of the invention.

Unless otherwise indicated, all tautomeric forms of the compounds of the invention are within the scope of the invention.

Additionally, unless otherwise indicated, structures depicted herein are also meant to include compounds that differ only in the presence of one or more isotopically enriched atoms. For example, compounds having the present structures except for the replacement of hydrogen by deuterium or tritium, or the replacement of a carbon by a $^{13}C$- or $^{14}C$-enriched carbon are within the scope of this invention. Such compounds are useful, for example, as analytical tools or probes in biological assays. Such compounds, especially deuterium analogues, can also be therapeutically useful.

The compounds of the invention are defined herein by their chemical structures and/or chemical names. Where a compound is referred to by both a chemical structure and a chemical name, and the chemical structure and chemical name conflict, the chemical structure is determinative of the compound's identity.

A further aspect of the invention describes antibodies raised against the immunogen described in the previously. The term "antibody" as used herein refers to an immunoglobulin or immunoglobulin-like molecule. In a preferred embodiment, the antibodies are monoclonal or polyclonal antibodies. However, the skilled person will understand that any type of immunoglobulin molecule or fragment thereof can be used, for example Fab fragments, scFv fragments and any other antigen binding fragments all of which fall within the scope of the current invention. The antibodies may be produced by any method as known to those skilled in the art. Any suitable host animal may be used in the immunisation process, preferably a mammalian animal for example, but not limited to, a sheep, rabbit, mouse, guinea pig or horse. In addition, the antibodies may be in the form of polyclonal antisera.

When used in reference to an antibody, the word "specific" or "specificity" in the context of the current invention refers to the analyte or analytes that are preferably bound by the antibody, as gauged by a suitable metric such as the cross-reactivity. For purposes of comparison, one analyte with high cross-reactivity is generally given a value of 100%, with all other analytes accorded a value relative to this; in addition, as is known by one skilled in the art, for cross-reactivity to be of practical use the analyte specific antibody must display a high sensitivity as measured by a suitable metric such as the $IC_{50}$. The $IC_{50}$ is a commonly used indicator of antibody sensitivity for immunoassays. To enable an assay to be effectively applied in the field, an $IC_{50}$ of less than or about 20 ng/ml, preferably less than or about 10 ng/ml, more preferably less than or about 5 ng/ml, and most preferably less than about 1 ng/ml for any individual analyte is preferred. Preferably the antibody of the current invention has an $IC_{50}$ of less than about 1 ng/ml for pregabalin. More preferably the $IC_{50}$ for pregabalin is about 0.266 ng/ml. The use of the word 'about' accounts for the expected minor variations in the measured $IC_{50}$ value which may arise during scientific analyses by different individuals when effecting the assay or from slight differences in assay equipment and reagents.

The phrase "an antibody which binds or specifically binds to an epitope of structure..." implies that the antibody, if polyclonal, will comprise clones whose high concentration and binding characteristics ensure an assay incorporating the antibody will bind to and ultimately support the identification of the compound of interest. Alternatively, the antibody could be a monoclonal antibody specific for a particular structural part of or the whole of the compound. There are several parameters that can be used to compare the relative degree of binding to an antibody of different analytes including the lowest limit of detection, the lowest limit of quantification and the $IC_{50}$. The $IC_{50}$ can be determined using a competitive assay and can be used to derive analyte cross-reactivities. Given the $IC_{50}$ of various analytes, their cross-reactivities, often represented as relative percentages, can be calculated.

The antibodies of the invention are further characterised in that they show no significant cross-reactivity with at least one, optionally at least five, further optionally all of the following compounds: 1,3-dimethylamylamine (DMAA), N-methyl pregabalin, acetylcholine, carbamazepine, ethosuximide, felbamate, gabapentin, gabapentin encarbil, gamma-aminobutyric acid, glutamic acid, lacosamide, lamotrigine, levetiracetam, oxcarbazepine, perampanel, phenobarbital, phenytoin, retigabine, rufinamide, serotonin, stiripentol, tiagabine, topiramate, valproic acid, vigabatrin and zonisamide.

The antibodies of the invention are further characterised in that compared to a cross-reactivity of 100% to pregabalin they show < about 5%, or < about 2.5% cross-reactivity with at least one, optionally at least five, further optionally all of the following compounds: 1,3-dimethylamylamine (DMAA), N-methyl pregabalin, acetylcholine, carbamazepine, ethosuximide, felbamate, gabapentin, gabapentin encarbil, gamma-aminobutyric acid, glutamic acid, lacosamide, lamotrigine, levetiracetam, oxcarbazepine, perampanel, phenobarbital, phenytoin, retigabine, rufinamide, serotonin, stiripentol, tiagabine, topiramate, valproic acid, vigabatrin and zonisamide. The antibodies of the invention are further characterised in that they show no significant cross-reactivity (for example, < about 1%) with the following compounds: acetylcholine, carbamazepine, ethosuximide, felbamate, gabapentin, gabapentin encarbil, gamma-aminobutyric acid, glutamic acid, lacosamide, lamotrigine, levetiracetam, oxcarbazepine, perampanel, phenobarbital, phenytoin, retigabine, rufinamide, serotonin, stiripentol, tiagabine, topiramate, valproic acid, vigabatrin and zonisamide. The terms "bind", "able to bind to" or "capable of binding" as used herein means that under standard immunoassay conditions, for example as described in 'Immunoassay: A practical guide' by Brian Law, Taylor and Francis Ltd (ISBN 0-203-48349-9), the antibodies will bind to said molecules. Due to inter-molecular attractive forces such as hydrogen bonding and van der Waal's forces there is often a degree of binding or affinity between two molecules whatever their respective structures; the skilled person recognizes that no cross-reactivity or minimal cross-reactivity implies that in the context of a working immunoassay any binding or interaction between an antibody and non-target analytes is at such a low level that it does not compromise the integrity of the immunoassay i.e. false positives are avoided.

In addition to or independently of these cross-reactivity profiles, it is preferable that the antibodies have an $IC_{50}$ of < about 1.00 ng/ml for pregabalin; more preferably the $IC_{50's}$ of the antibodies are < about 0.50 ng/ml for pregabalin, more preferably the $IC_{50's}$ of the antibodies are about 0.266 ng/ml for pregabalin. The sensitivities of the antibodies can be measured by any suitable metric used in the art such as the limit of detection, limit of quantitation as well as the $IC_{50}$.

The invention also describes a competitive immunoassay method of detecting or determining the antiepileptic drug pregabalin in an *in vitro* sample taken from an individual, (i.e. a subject) comprising; contacting the sample with one or more detecting agents and one or more antibodies of the invention; detecting, or determining the quantity of the one or more detecting agents; and deducing from calibrators the presence of, or amount of, pregabalin in the sample. In a preferred embodiment the one or more antibodies are characterised as having been generated from an immunogen of the invention. By 'detecting' is meant qualitatively analysing for the presence or absence of a substance; by 'determining' is meant quantitatively analysing for the amount of substance present. It is common practice that in the immunoassay method the presence or amount of target analyte(s) (in the current instance pregabalin) is gauged by reference to one or more calibrator values usually in the form of a cut-off value or calibration curve; the Immunoassay Development section herein describes the use of a calibrator to construct a calibration curve or 'standard curve' which allows the sensitivity (in this case the $IC_{50}$) and cross-reactivity of antibodies to the target analytes to be derived.

A calibrator is well known in the art and refers to a reference value or values, the reference being a substance which enables a threshold concentration or the exact or calibrator equivalent amount of analyte(s) to be determined. The determination of an exact or calibrator equivalent amount of analyte(s) usually requires the construction of a calibration curve (also known as a standard curve). The number of calibrator points can vary, but is usually from 5 to 9. To enable a practical assay for clinical/commercial use, the binding of the antibody to the analyte(s) must be such that the concentration at which the analytes are detected or determined is at an acceptable level. The detecting agent (also known as a tracer or conjugate) is the substance which emits a detectable signal and comprises a moiety of similar structure to a target analyte conjugated, by way of a crosslinker, to a labelling agent, that is able to bind to one of the antibodies of the invention.

The term "subject" refers to an animal (e.g., a bird such as a chicken, quail or turkey, or a mammal), specifically a "mammal" including a non-primate (e.g., a cow, pig, horse, sheep, rabbit, guinea pig, rat, cat, dog, and mouse) and a primate (e.g., a monkey, chimpanzee and a human), and more specifically a human. In one embodiment, the subject is a non-human animal such as a farm animal (e.g., a horse, cow, pig or sheep), or a pet (e.g., a dog, cat, guinea pig or rabbit). In another embodiment, the subject is a "human".

The term "sample" includes "biological sample", which, as used herein, includes, without limitation, cell cultures or extracts thereof; biopsied material obtained from a mammal or extracts thereof; blood, saliva, urine, faeces, semen, tears, or other body fluids or extracts thereof. In one embodiment, the biological sample is a peripheral biological fluid, but is including whole blood, serum, plasma, hair or urine. The sample may also be a solution which is suspected of containing a drug. The solution can be a liquid suspected of containing one or more of these drugs. Samples may require pre-treatment to achieve a formulation suitable for analysis, such as dissolution in a suitable liquid. An *in vitro* sample is any suitable biological sample such as, but not limited to, blood, serum, plasma, urine or saliva. The *in vitro* sample is preferably a serum, plasma or urine sample. The immunoassay method is most suited to the competitive assay format in which a target analyte which binds to the antibody i.e. the molecule to be detected or determined, competes with a detecting agent (also called a 'tracer' or 'conjugate') which also binds to the antibody, for binding sites on the antibody; the more analyte present the less detecting agent that binds to the antibody and the lower the measured signal. The detecting agent can be a substance such as an enzyme, a substance having fluorescent properties or a radioactive

label; it is usual for an immunoassay that the detecting agent is a structure similar to the target analyte in which an enzyme or a substance having fluorescent properties has been conjugated, or in which a radiolabel has been incorporated. Conjugation is by way of standard methods familiar to the skilled person. Typically enzymes promote light emission from substrates added to the assay medium. The 'detecting and determining' criteria for the immunoassay method includes, as is well-known in the art, detection of a signal, exceeding a pre-defined cut-off/concentration value or measuring the calibrator equivalent value as derived from a calibrator curve (also referred to as a standard curve). In relation to the antibodies described herein, in the context of the current invention, 'from which they are raised' is synonymous with 'to which they are derived' or 'from which they are generated'. The antibodies of the invention, separate of or as part of the immunoassay method described, do not display cross-reactivity to any compounds which could undermine the immunoassay method.

The detecting agent is a small molecule (generally of similar structure to a molecule to be detected), conjugated to a labelling agent that is able to bind to one of the antibodies of the invention. Alternative names for the detecting agent are the conjugate or tracer. The labelling agent is selected from an enzyme, a luminescent substance, a radioactive substance, or a mixture thereof. Preferably, the labelling agent is an enzyme, preferably a peroxidase, most preferably horseradish peroxidase (HRP). Alternatively, or additionally, the luminescent substance may be a bioluminescent, chemiluminescent or fluorescent material. Preferably, for the immunoassay method of the invention, the detecting agent is based on a compound with a pregabalin substructure conjugated to an enzyme or fluorescent molecule. An example detecting agent of the current invention is hapten A conjugated to HRP (see Example 9).

The invention further describes a substrate with which the antibodies of the invention engage. The antibodies can engage with the substrate by, for example, passive adsorption or can be chemically bonded to the substrate attached by way of, for example, covalent bonds. Such covalent bonding generally requires the initial introduction of a chemically active compound covalently attached to the substrate surface prior to antibody addition. The antibody itself may also require the addition of a chemical activating group to achieve substrate bonding. These requirements are well known in the art. The substrate can be any medium capable of adsorbing or bonding to an antibody, for example a bead or nanoparticle (optionally chemically-activated), but is preferably of a planar conformation (optionally chemically-activated) such as a microtitre plate (as in Example 10 below) or a biochip.

Microtitre plates (or microplates) commonly consist of 6, 24, 96, 384 or 1536 sample wells arranged in a 2:3 rectangular matrix. 96 well microtitre plates are commonly used in an ELISA. A biochip is a thin, wafer-like substrate with a planar surface which can be made of any suitable material such as glass or plastic but is preferably made of ceramic. The biochip is able to be chemically-activated prior to antibody bonding or is amenable to the passive adsorption of antibodies. The skilled person in biochip development for immunoassay application will recognize that a planar surface at high resolution e.g. if using a scanning electron microscope (SEM), is not perfectly 'flat' but will possess an uneven surface, the important aspect being that the 'approximately' planar surface is suitable for application. A microlayer coating of material can optionally be added to the planar surface of the substrate prior to antibody immobilisation. Either the upper surface or both surfaces of the substrate can be coated.

The invention further describes a kit comprising an antibody of the invention. The antibody is preferably attached to a microtiter plate or biochip; the kit can also incorporate a tracer and any further reagents necessary to enable a detectable or measurable signal to be produced. The kit can be used for therapeutic drug monitoring of pregabalin, particularly in patients with renal failure, which may include, for example, determining effective dosages for individual patients and assessing adherence to therapy.

Enzyme immunoassays, ELISAs

The enzyme-linked immunosorbent assay (ELISA) is a test that uses antibodies and colour change to identify a substance.

Antigens from the sample are attached to a surface. Then, a further specific antibody is applied over the surface so it can bind to the antigen. This antibody is linked to an enzyme, and, in the final step, a substance containing the enzyme's substrate is added. The subsequent reaction produces a detectable signal, most commonly a colour change in the substrate.

Performing an ELISA involves at least one antibody with specificity for a particular antigen. The sample with an unknown amount of antigen is immobilized on a solid support (usually a polystyrene microtiter plate) either non-specifically (via adsorption to the surface) or specifically (via capture by another antibody specific to the same antigen, in a "sandwich" ELISA). After the antigen is immobilized, the detection antibody is added, forming a complex with the antigen. The detection antibody can be covalently linked to an enzyme, or can itself be detected by a secondary antibody that is linked to an enzyme through bioconjugation. Between each step, the plate is typically washed to remove any proteins or antibodies that are not specifically bound. After the final wash step, the plate is developed by adding an enzymatic substrate to produce a visible signal, which indicates the quantity of antigen in the sample.

Lateral flow devices

In recent years, the in vitro diagnostics industry has made enormous efforts to develop immunochromatographic tests. Such tests have found applications in both clinical and non-clinical fields. A clinical utility of this test format is particularly suited to point of care utilities.

Rapid immunochromatographic test devices, e.g. in the form of a test strip, are made up of a number of components. Such a test strip commonly includes a sample pad, a conjugate pad, a membrane, e.g. a nitrocellulose membrane, and an absorbent pad. The membrane is usually attached by means of an adhesive to a supporting backing, e.g. made of plastic. In practice, the user dispenses a patient sample (such as urine or whole blood) onto the sample pad. The sample then flows through the sample pad into the conjugate pad, where it mixes with, and releases, the detector reagent. This mixture then flows across the membrane, where it binds with the test and control reagents located in the capture test zone (sample zone) and negative control zone, respectively. When the mixture binds to the reagent that forms the test line, a positive result is indicated. The colour intensity of the test line is proportional to the concentration of analyte in the sample. Excess sample that flows beyond the test and control zones is taken up in the absorbent pad.

Rapid immunochromatographic test devices for diagnostic purposes are easy to operate and thus do not only contribute to the comfort of professional users, e.g. medical stuff, but also allow the operation by non-professionals users, e.g. most patients.

Biochips

Biochips are components used for example in chemical analysis (including Proteomic and Molecular analysis) either to host a test reaction and/or to supply samples under test or reagents. Generally, a Biochip comprises a solid substrate, on which is arranged one or more test sites at which a reaction can take place in use. For instance, the test site may carry one or more reagents (e.g. ligands such as antibodies or antigens) adsorbed to the substrate, which are activated by the addition of a sample substance (e.g. analytes present in the sample bind to specific ligands). Such chips are sometimes referred to as a "lab on a chip" and can also incorporate tools for controlling steps of a reaction. As an example, one Biochip supplied by Randox Laboratories Limited (55 Diamond Road, Crumlin, County Antrim, United Kingdom, BT29 4QY) is used as a medium for performing multiplex analysis of biological samples using a chemiluminescence method. In this example, the Biochip takes the form of a small ceramic chip with a specialised surface preparation which is sensitive to environmental degradation. Therefore the Biochip is generally delivered in an environmentally sealed format, usually evacuated, sealed foil bags.

For instance, the Evidence™ analyser by Randox Laboratories Ltd uses biochips which are fitted into a plastic holder defining three recesses arranged in a line. Each recess is approximately square and sized to just accommodate a biochip, which is also square, with a small clearance to allow the chip to be placed. The "strip" of three mounted biochips is placed within a sealed foil bag for storage, which is then opened when the biochips are required for use. The plastic holder may be placed on a carrier alongside two further strips of three biochips to form a 3x3 array of biochips. The carrier has a keying feature for engagement with a robotic arm such that the array can be transported within the analyser via robotic handling. This configuration is useful for batch analysis.

A "Biochip" is a general term for a reaction platform for hosting chemical, biochemical, proteomic or molecular tests, as may be required for medical diagnosis, drug detection, etc. Typically, a Biochip comprises an inert substrate, such as silicon or glass (often of the order of about 1 $cm^2$ or less in surface area), on which one or a plurality of reaction sites is provided. The sites generally carry one or more ligands, for example, one or more antibodies, selected for the test (or "assay") to be performed, adsorbed to the surface of the chip for activation upon combination with a sample applied to the chip (e.g. a blood sample) and/or a reagent. The reactions can be detected using a number of alternative techniques, including detection of chemiluminescence generated by the reaction. Some biochips carry a very large number (hundreds or thousands) of such tests sites, typically arranged in a grid or array, making it possible to carry out numerous assays simultaneously, and using the same single specimen.

General methodology

*Preparation of Haptens, Immunogens and Detecting Agents*

Although haptens provide defined structural epitopes, they are not in themselves immunogenic and therefore need to be conjugated to carrier materials, which will elicit an immunogenic response when administered to a host animal. Appropriate carrier materials commonly contain poly(amino acid) segments and include polypeptides, proteins and protein fragments. Illustrative examples of useful carrier materials are bovine serum albumin (BSA), egg ovalbumin (OVA), bovine gamma globulin (BGG), bovine thyroglobulin (BTG), keyhole limpet haemocyanin (KLH) etc. Alternatively, synthetic poly(amino acids) having a sufficient number of available amino groups, such as lysine, may be employed, as

may other synthetic or natural polymeric materials bearing reactive functional groups. Also, carbohydrates, yeasts or polysaccharides may be conjugated to the hapten to produce an immunogen. The haptens can also be coupled to a detectable labelling agent such as an enzyme (for example, horseradish peroxidase), a substance having fluorescent properties or a radioactive label for the preparation of detecting agents for use in the immunoassays. The fluorescent substance may be, for example, a monovalent residue of fluorescein or a derivative thereof. Immunogen formation for the invention described herein involves conventional conjugation chemistry. In order to confirm that adequate conjugation of hapten to carrier material has been achieved, prior to immunisation, each immunogen is evaluated using matrix-assisted UV laser desorption /ionisation time-of-flight mass spectroscopy (MALDI-TOFMS).

*General Procedure for MALDI-TOF Analysis of Immunogens.*

MALDI-TOF mass spectrometry can be performed using a Voyager STR Biospectrometry Research Station laser-desorption mass spectrometer coupled with delayed extraction. An aliquot of each sample to be analysed can be diluted in 0.1 % aqueous trifluoroacetic acid (TFA) to create 1 mg/ml sample solutions. Aliquots (1 μl) can be analysed using a matrix of sinapinic acid and bovine serum albumin (Sigma-Aldrich) as an external calibrant.

*Preparation of Antisera*

In order to generate polyclonal antisera, 2 mg of an immunogen of the present invention is prepared in PBS, mixed at a ratio of 50 % immunogen in PBS with 50% Freund's Complete adjuvant (Sigma, Product Number - F5881) and emulsified by repeatedly passing the mixture through a tip on the end of a 1 ml syringe, until it reaches the required semi-solid consistency. 1 ml of the mixture is then injected into a host animal, such as rabbit, sheep, mouse, guinea pig or horse. Sheep are the preferred host animal. Further injections (boosts) are administered on a monthly basis (1 mg of immunogen is prepared in PBS and mixed at a ratio of 50 % immunogen in PBS with 50 % of Freund's Incomplete Adjuvant, Sigma product Number - F5506) until the required titre is achieved. Serum is sampled for evaluation of the antibody titre.

Briefly, blood is collected by applying pressure to the exposed jugular vein and inserting a clean 14 gauge hypodermic needle to remove 500 ml of blood per sheep, under gravity. The blood is stored at 37 °C for a minimum of 1 hour before the clots are separated from the side of the centrifuge bottles using disposable 1 ml pipettes (ringing). The samples are stored at 4 °C overnight.

Samples are then centrifuged at 4200 rpm for 30 minutes at 4 °C. The serum is poured off and centrifuged again, at 10,000 rpm for 15 minutes at 4 °C, before being aliquoted and stored at <-20 °C.

The Immunoglobulin (Ig) fraction is extracted from the antisera via caprylic acid/ammonium sulphate precipitation of immunoglobulin.

The antibody titre is evaluated by coating a microtiter plate (Thermo Fisher Scientific NUNC, 468667) with antibody (125 μl/well) in coating buffer (10 mM Tris pH 8.5) at 37 °C for 2 hours. The plate is then washed 4 times over 10 minutes with working strength mixture of Tris-Buffered Saline and Tween 20 (TBST). 50 μl of sample/standard (pregabalin) is added to the appropriate wells in triplicate, followed by 75 μl of hapten-HRP conjugate and incubated at 25 °C for 1 hour. The plate is then washed and 125 μl of TMB (Randox Laboratories, 4380-15) added to each well and left at room temperature for 20 mins in the dark. The reaction is stopped using 125 μl of 0.2 M sulphuric acid. The absorbances are read at 450 nm with an ELISA microplate reader (BIO-TEK Instruments, Elx800) and the means calculated. Antibody sensitivity can then be determined.

When the optimal titre has been attained, the host animal is bled to yield a suitable volume of specific antiserum (overall this results in 20 bleeds in total, with approximately 200 ml of antiserum achieved per bleed). The degree of antibody purification required depends on the intended application. For many purposes, there is no requirement for purification, however, in other cases, such as where the antibody is to be immobilised on a solid support, purification steps can be taken to remove undesired material and eliminate non-specific binding.

Various purification steps are available if required, including Immunoglobulin Precipitation (as described above), Antigen-specific affinity purification, Size-exclusion chromatography and Ion Exchange Chromatography.

*Immunoassay Development*

The process of developing an immunoassay is well known to the person skilled in the art. Briefly, for a competitive immunoassay in which the target analyte is a non-immunogenic molecule such as a hapten, the following process is conducted: antibodies are produced by immunizing an animal, preferably a mammalian animal, by repeated administration of an immunogen. The serum from the immunized animal is collected when the antibody titre is sufficiently high. A detecting agent is added to a sample containing the target analyte and the raised antibodies, and the detecting agent and analyte compete for binding to the antibodies. The process may comprise fixing said serum antibodies to a backing

substrate such as a polystyrene solid support or a ceramic chip. The antibodies can be polyclonal or monoclonal antibodies.

This can be carried out using an ELISA based format as described above for measuring antibody titre or as a Biochip based format. Details of how the antibodies are fixed to the Biochip are described in FitzGerald, S. P. et al, Clin. Chem. 51(7); 1165-1176; 2005. The signal emitted in the immunoassay is proportionate to the amount of detecting agent bound to the antibodies which in turn is inversely proportionate to the analyte concentration. The signal can be detected or quantified by comparison with a calibrator.

Examples

Numbers in (bold) refer to structures in figures 3-6.

Example 1: Preparation of N-Boc pregabalin (1)

To a solution of Pregabalin (2.0 g, 0.012 moles) in dioxane (20 ml.) was added sodium hydroxide (NaOH) solution (1N) followed by $Boc_2O$ (3.3 g, 0.015 moles, 1.2 eq). The resulting mixture was stirred at room temperature while being monitored and maintained at a value of pH 9-10 by adding NaOH solution (1N) drop-wise. When the pH of the reaction mixture remained constant the stirring was continued for further 2 hours when thin layer chromatography (TLC) showed completion of reaction. The solvents were removed in vacuum and the aqueous solution was acidified to pH 2-3 with HCI solution (1M) and extracted with ethyl acetate (3x50 ml). The combined organics were washed with water (50 ml), brine (50 ml), dried over sodium sulphate, filtered and concentrated to dryness to give 3.1 g of N-Boc pregabalin (1) as a foamy white solid.

Example 2: Preparation of N-Boc pregabalin aminoethylmaleimide (2)

To a solution of N-Boc pregabalin (1) (500 mg, 1.93 mmol) in dimethylformamide (DMF) (10 ml.) were added 2-(1H-benzotriazol-1-yl)-1,1,3,3-tetramethyluronium hexafluorophosphate (HBTU) (877 mg, 2.3 mmol, 1.2 eq), HOBt (354 mg, 2.3 mmol, 1.2 eq) and ethylene dichloride (EDC) (444 mg, 2.31 mmol, 1.2 eq) and the resulting mixture was stirred at room temperature for 3 hours. Triethylamine (322 μl, 2.3 mmol, 1.2 eq) was added followed by 2-aminoethyl maleimide hydrobromide (588 mg, 2.3 mmol, 1.2 eq) and the reaction mixture was stirred at room temperature for another 2 hours when TLC showed completion of reaction. The solvent was removed in vacuum and the residue was triturated with ethyl acetate when most of the by-products precipitated and removed by filtration. The solvent was removed under vacuum and the resulting residue was purified by column chromatography (silica gel, 20-50-100% ethyl acetate in hexane) to give (287 mg) of N-Boc pregabalin aminoethylmaleimide (2).

Example 3: Preparation of Pregabalin aminoethylmaleimide (Hapten A)

To a solution of N-Boc pregabalin aminoethyl maleimide (2) (287 mg, 0.75 mmol) in anhydrous dichloromethane (1 ml) was added trifluoroacetic acid (TFA) (1 ml) at 0 °C. The reaction mixture was allowed to slowly warm up at room temperature and was stirred overnight. The solvent was removed under vacuum and the residue was purified by column chromatography (silica gel, 0-10% methanol in chloroform) to give 267 mgs Pregabalin aminoethylmaleimide (Hapten A) as a sticky oil.

NMR 13C: 172.47, 171.91, 135.39, 51.66, 43.57, 40.87, 39.93, 38.44, 37.77, 23.52 and 23.03

Reference Example 4: Preparation of N-Boc pregabalin acetylthiopropioanamido (**3**)

To a solution of N-Boc pregabalin (**1**) (500 mg, 1.93 mmol) in DMF(10 ml) was added HBTU (877 mg, 2.3 mmol, 1.2 eq), hydroxybenzotriazole (HOBt) (354 mg, 2.3 mmol, 1.2 eq) and EDC(444 mg, 2.31 mmol, 1.2 eq) and the resulting mixture was stirred at room temperature for 2 hours. Triethylamine (322 μl, 2.3 mmol, 1.2 eq) was added followed by the addition 3-acetylthiopropylamine (308 mg, 2.3 mmol, 1.2 eq) and the reaction mixture was stirred at room temperature for another 1 hour when TLC showed completion of reaction. The solvent was removed under vacuum and the residue was triturated with ethyl acetate when most of the by-products precipitated and were removed by filtration. The solvent was removed under vacuum and the resulting residue was purified by column chromatography (silica gel, 20-50-100% ethyl acetate in hexane) to give 346 mgs of N-Boc pregabalin acetylthiopropianimido (**3**)

Reference Example 5: Preparation of Pregabalin acetylthioprapanamine (Hapten B)

To a solution of N-Boc pregabalin-acetylthiopropianimido (**3**) (346 mg, 0.92 mmol) in 1 ml anhydrous dichloromethane was added 1 ml TFA at 0 °C. The reaction mixture was allowed to slowly warm up at room temperature and was stirred for 5 hours. The solvent was removed by vacuum and the residue was purified by column chromatography (silica gel, 0-10% methanol in chloroform) to give 302 mg Pregabalin 3- acetylthiopropane-1-amide (Hapten B) as a yellow oil.

Reference Example 6: Preparation of Pregabalin N-acetylthiopropanamide (Hapten C)

To a solution of Pregabalin (500 mg, 3.14 mmol) in anhydrous DMF (5 ml) were added di-isopropyl ethylamine (DIEPA) (591 μl, 3.45 mmol, 1.1 eq) and SATP-NHS (N-succinimidyl-S-acetylthiopropionate-N-Hydroxysuccinimide) (847 mg, 3.45 mg, 1.1 eq) and the reaction mixture was stirred at room temperature overnight. The solvent was evaporated under vacuum and the residue was purified by column chromatography (silica gel, 0-10% methanol in chloroform) to give 500 mgs of Pregabalin N-acetylthiopropanamide (Hapten C) as a white solid.

Example 7: Conjugation of Hapten A to BSA (Immunogen I)

Preparation of 2-Iminothiolane modified BSA: 2-Iminothiolane hydrochloride solution (0.042 ml @ 40 mg/ml Phosphate Buffered Saline, pH 8.0 with 100 mM ethylene diamine tetraacetic acid (EDTA)) was added drop-wise to carrier protein BSA (20 mg) dissolved in Phosphate Buffered Saline, pH 8.0 with 100 mM EDTA (1 ml) while stirring. And then the resulting solution was rolled at room temperature overnight. Excess 2-Iminothiolane hydrochloride was removed by dialysis at 4 °C against PBS, pH 8.0 with 2.0 mM EDTA.

Preparation of Immunogen I: Pregabalin aminoethylmaleimide (Hapten A) (3.47 mg) dissolved in DMF (0.2 ml) was added to 2-Iminothiolane modified BSA while stirring, then the resulting solution was rolled overnight at room temperature, and then N-Ethylmaleimide (3 mg) dissolved in DMF (0.06 ml) was added, rolled for two hours at RT. The excess of the Hapten A and N-Ethylmaleimide were removed by dialysis against PBS, pH 7.2 to give Immunogen I.

MALDI results showed 8.5 molecules of Hapten A had been conjugated to one molecule of BSA.

Example 8: Conjugation of Hapten A to KLH (Immunogen II)

Preparation of 2-Iminothiolane modified KLH: 2-Iminothiolane hydrochloride solution (0.212 ml @ 40 mg/ml Phosphate Buffered Saline, pH 8.0 with 100 mM EDTA) was added drop-wise to carrier protein KLH (100 mg) dissolved in Phosphate Buffered Saline, pH 8.0 with 100 mM EDTA (5 ml) while stirring. And then the resulting solution was rolled at room temperature overnight. Excess 2-Iminothiolane hydrochloride was removed by dialysis at 4 °C against PBS, pH 8.0 with 2.0mM EDTA.

Preparation of Immunogen II: Pregabalin aminoethylmaleimide (Hapten A) (17.33 mg) dissolved in DMF (1.0 ml) was added to 2-Iminothiolane modified KLH while stirring, then the resulting solution was rolled overnight at room temperature, and then N-Ethylmaleimide (15.4 mg) dissolved in DMF (0.3 ml) was added, rolled for two hours at RT. The excess of the Hapten A and N-Ethylmaleimide were removed by dialysis against PBS, pH 7.2 to give Immunogen II.

Example 9: Conjugation of Hapten A to HRP (Tracer 1)

Preparation of 2-Iminothiolane modified HRP: 2-Iminothiolane hydrochloride solution (0.05 ml @ 6.25 mg/ml in 50 mM Phosphate Buffered Saline, pH 8.0 with 2.0 mM EDTA) was added drop-wise to HRP (20 mg) dissolved in 50 mM Phosphate Buffered Saline, pH 8.0 with 100mM EDTA (1.0 ml) while stirring. And then the resulting solution was rolled at room temperature overnight. Excess 2-Iminothiolane hydrochloride was removed with PD-10 column (Pharmacia), pre-equilibrated with 50 mM PBS pH 8.0 with 2.0 mM EDTA. Keep the solution in the dark during the conjugation.

Preparation of the HRP-Hapten A: Pregabalin aminoethylmaleimide (Hapten A) (2.0 mg) dissolved in DMF (0.2 ml) was added to 2-Iminothiolane modified HRP while stirring, then the resulting solution was rolled overnight at room temperature, and then N-Ethylmaleimide (3.0 mg) dissolved in DMF (0.3 ml) was added, rolled for one hours at RT. The excess of the Hapten A and N-Ethylmaleimide were removed with PD-10 column (Pharmacia), pre-equilibrated with 50 mM PBS pH 8.0 with 2.0 mM EDTA followed by dialysis against PBS, pH 7.2 to give the tracer HRP conjugate to Hapten-A (Tracer 1).

Example 10: Development of an immunoassay for pregabalin

IgG was extracted from the antisera using ammonium sulphate/caprylic acid precipitation and the purified antibody was immobilised on a 96 well ELISA plate at 5 μg/ml in 10 mM TRIS buffer, pH 8.5 overnight at 4 °C. The assay was based on competition for binding sites of a polyclonal antibody between HRP tracer and pregabalin or potential cross-reactants. The plate was washed (x3) with TBST and the calibrator (pregabalin) or potential cross reactants added

(50 μl per well), followed by HRP tracer (75 μl/well) to the appropriate wells. The plates were then incubated for 60 minutes at 25 °C. They were then subjected to 2 quick wash cycles using TBST, followed by 4 x 2 minute wash cycles. 125 μL of signal 25 (TMB) was then added to each well for 20 mins at room temperature in the dark. The reaction was stopped by the addition of 125 μl of 0.2 M Sulphuric Acid per well and the plates read immediately at 450 nm. Calibration curves were generated and these were used to determine the sensitivity and specificity of the immunoassay for pregabalin and potential cross-reactants. The data was inputted to a computer program called 'KC Junior' (Biotek). It gives a 4 parameter fit curve and allows the calculation of concentrations between the standard runs. This program is used to calculate the $IC_{50}$ values by dividing the 0 ng/ml optical density (OD) value by 2 and obtaining the concentration value from the curve for this OD. The results of this study are presented in Table 1 and Table 2 shows compounds tested which had no significant cross-reactivity with antibodies of the invention, cross-reactivity being calculated according to the following formula:

$$\%CR = IC_{50} \text{ pregabalin}/ IC_{50} \text{ CR X } 100$$

Wherein %CR is the percentage cross-reactivity, $IC_{50}$ pregabalin is the concentration of pregabalin which causes 50% displacement of signal and $IC_{50}$ CR is the concentration of potential cross-reactant that causes 50% displacement of signal.

The antibody (RS2845) used to generate the data in the tables below was raised from immunogen II (Example 8) and the HRP tracer was prepared as in Example 9 (Tracer 1). All of the compounds in table 2 were tested at 5 μg/ml.

**Table 1**, ELISA results for antibody RS2845 (raised against Immunogen II of Example 8) coated at 5 μg/ml with HRP labelled tracer (Tracer 1 of Example 9) at a dilution of 1/5k using pregabalin as a calibrator.

| Pregabalin (ng/ml) | Average OD | $B/B_0$ |
|---|---|---|
| 0.00 | 2.446 | 100 |
| 0.02 | 1.862 | 76 |
| 0.08 | 1.276 | 52 |
| 0.31 | 0.696 | 28 |
| 1.25 | 0.346 | 14 |
| 5.00 | 0.178 | 7 |
| $IC_{50}$ | 0.266 | |
| B = absorbance at 450nm at x ng/ml standard concentration<br>$B_0$= absorbance at 450nm at 0 ng/ml standard concentration<br>$IC_{50}$ = standard concentration which produces 50% $B/B_0$<br>OD= Optical density | | |

6 different negative blood and urine samples were run diluted 1 in 10 in TBST + 1% ProClin® (Sigma Aldrich) and produced the following limits of detection (LOD's):
LOD calculated as the mean +2STDEV:

Blood - 0.424μg/ml
Urine - 0.233μg/ml

**Table 2,** Compounds showing no significant cross-reactivity with for antibody RS2845 (raised against Immunogen II of Example 8).

| | |
|---|---|
| Acetylcholine | Perampanel |
| Carbamazepine | Phenobarbital |
| Ethosuximide | Phenytoin |
| Felbamate | Retigabine |
| Gabapentin | Rufinamide |

(continued)

| | |
|---|---|
| Gabapentin encarbil | Serotonin |
| Gamma-aminobutyric acid | Stiripentol |
| Glutamic acid | Tiagabine |
| Lacosamide | Topiramate |
| Lamotrigine | Valproic acid |
| Leveti racetam | Vigabatrin |
| xcarbazepine | Zonisamide |

**Table 3,** Cross Reactivity of various cross reactants for antibody RS2845 (raised against Immunogen II of Example 8) coated at 5 μg/ml with HRP labelled tracer (Tracer 1 of Example 9) at a dilution of 1/5k using pregabalin as a calibrator. All cross-reactants were assessed at 5 μg/ml.

| Cross-Reactivity of Pregabalin compounds | |
|---|---|
| Compound | % Cross-Reactivity |
| Pregabalin | 100 |
| 1,3-dimethylamylamine (DMAA) | 2.2 |
| Vigabatrin | <l |
| Felbamate | <l |
| Retigabine | <l |
| Lacosamide | <l |
| Stiripentol | <l |
| Acetylcholine Cl | <l |
| D-Glutamic Acid | <l |
| L-Glutamic Acid | <l |
| Tiagabine HCl | <l |
| Gamma-Aminobutyric Acid | <l |
| N-methylpregabalin | <l |
| Valporic Acid | <l |
| Leveti racetam | <l |
| Topiramate | <l |
| Serotonin | <l |
| Carbamazepine | <l |
| Gabapentin | <l |
| Phenobarbital | <l |

**Claims**

**1.** An immunogen consisting of 'Hapten A'

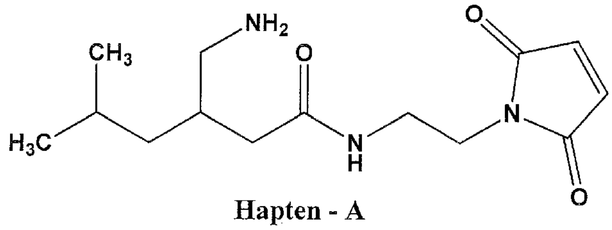

Hapten - A

conjugated to an antigenicity-conferring carrier material (accm), wherein the accm is coupled to the maleimide moiety of Hapten A and is selected from the group consisting of bovine serum albumin (BSA) and keyhole limpet haemocyanin (KLH).

2. An antibody raised against an immunogen of Claim 1 , **characterised in** having an $IC_{50}$ of less than 1 ng/ml for pregabalin.

3. The antibody of Claim 2 further **characterised in** having a cross-reactivity of 100% to pregabalin and a cross-reactivity of < 5% to at least one of 1,3-dimethylamylamine (DMAA), Vigabatrin, Felbamate, Retigabine, Lacosamide, Stiripentol, Acetylcholine, D-Glutamic Acid, L-Glutamic Acid, Tiagabine HCl, Gamma-Aminobutyric Acid, N-methylpregabalin, Valporic Acid, Levetiracetam, Topiramate, Serotonin, Carbamazepine, Gabapentin, and Phenobarbital.

4. A method of detecting or determining pregabalin in a sample comprising the steps of:

   i) Contacting the sample with one or more detecting agents and one or more antibodies of Claims 2 or 3;
   ii) Measuring the signal produced by the one or more detecting agents; and
   iii) Detecting or determining the presence of, or amount of pregabalin.

5. The method of Claim 4, wherein the antibody is adsorbed on or attached to a solid-state device.

6. The method of Claim 5, wherein the solid-state device is a biochip.

7. The method of Claim 6, wherein the solid-state device is a microtiter plate.

8. A kit for detecting or determining pregabalin in a sample, said kit comprising an antibody of Claims 2 or 3.

9. The kit of Claim 8, wherein the antibody is adsorbed on or attached to a solid-state device.

10. The kit of Claim 9, wherein the solid-state device is a microtiter plate or biochip

**Patentansprüche**

1. Immunogen bestehend aus 'Hapten A',

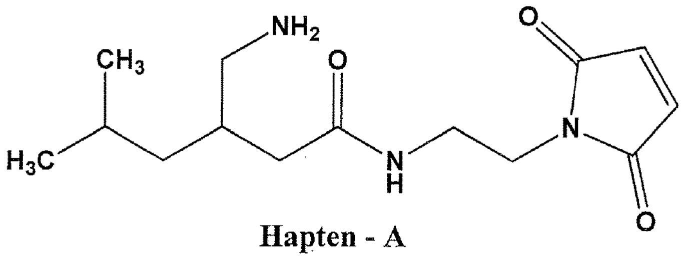

Hapten - A

konjugiert an ein Antigenität verleihendes Trägermaterial (accm), wobei das Accm an die Maleimid-Einheit von Hapten A gekoppelt ist und ausgewählt ist aus der Gruppe bestehend aus Rinderserumalbumin (BSA) und Keyhole-Napfschnecken-Hämocyanin (KLH).

2. Ein Antikörper gegen ein Immunogen nach Anspruch 1, **dadurch gekennzeichnet, dass** er eine IC50 von weniger als 1 ng/ml für Pregabalin aufweist.

3. Antikörper nach Anspruch 2, ferner **dadurch gekennzeichnet, dass** er eine Kreuzreaktivität von 100 % mit Pregabalin und eine Kreuzreaktivität von < 5 % mit mindestens einem von 1,3-Dimethylamylamin (DMAA), Vigabatrin, Felbamat, Retigabin, Lacosamid, Stiripentol, Acetylcholin, D-Glutaminsäure, L-Glutaminsäure, Tiagabin HCl, Gamma-Aminobuttersäure, N-Methylpregabalin, Valporinsäure, Levetiracetam, Topiramat, Serotonin, Carbamazepin, Gabapentin und Phenobarbital.

4. Verfahren zum Nachweis oder zur Bestimmung von Pregabalin in einer Probe, umfassend die Schritte:

   i) Inkontaktbringen der Probe mit einem oder mehreren detektionsmitteln und einem oder mehreren Antikörpern nach Anspruch 2 oder 3;
   ii) Messen des Signals, das von dem einen oder den mehreren detektionsmitteln erzeugt wird; und
   iii) Nachweis oder Bestimmung des Vorhandenseins oder der Menge von Pregabalin.

5. Verfahren nach Anspruch 4, wobei der Antikörper an einer Festkörper vorrichtung adsorbiert oder daran gebunden ist.

6. Verfahren nach Anspruch 5, wobei die Festkörper vorrichtung ein Biochip ist.

7. Verfahren nach Anspruch 6, wobei die Festkörper vorrichtung eine Mikrotiterplatte ist.

8. Ein Kit zum Nachweis oder zur Bestimmung von Pregabalin in einer Probe, wobei der Kit einen Antikörper nach Anspruch 2 oder 3 umfasst.

9. Kit nach Anspruch 8, wobei der Antikörper an einer Festkörper vorrichtung adsorbiert oder daran befestigt ist.

10. Kit nach Anspruch 9, wobei die Festkörper vorrichtung eine Mikrotiterplatte oder ein Biochip ist

**Revendications**

1. Un immunogène constitué de « Hapten A »

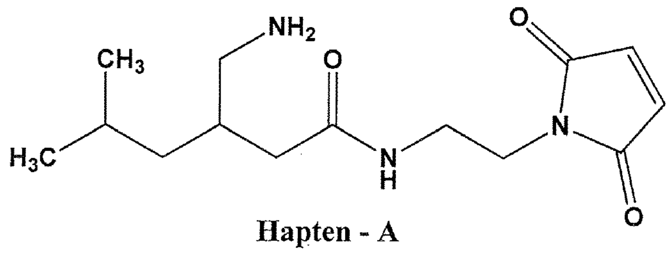

**Hapten - A**

conjugué à un matériau support conférant une antigénicité (accm), dans lequel l'accm est couplé à la fraction maléimide de l'haptène A et est choisi dans le groupe constitué de la sérumalbumine bovine (BSA) et de l'hémocyanine de patelle (KLH).

2. Anticorps dirigé contre un immunogène selon la revendication 1, **caractérisé en ce qu'** il a une CI50 inférieure à 1 ng/ml pour la prégabaline.

3. Anticorps selon la revendication 2, **caractérisé en outre en ce qu'**il a une réactivité croisée de 100 % avec la prégabaline et une réactivité croisée de < 5 % avec au moins l'un des 1,3-diméthylamylamine (DMAA), Vigabatrin, Felbamate, Retigabine, Lacosamide, Stiripentol, Acétylcholine, Acide D-Glutamique, Acide L-glutamique, chlorhydrate de tiagabine, acide gamma-aminobutyrique, N-méthylprégabaline, acide valporique, lévétiracétam, topiramate, sérotonine, carbamazépine, gabapentine et phénobarbital.

4. Méthode de détection ou de détermination de la prégabaline dans un échantillon comprenant les étapes de :

i) mise en contact de l'échantillon avec un ou plusieurs agents de détection et un ou plusieurs anticorps des revendications 2 ou 3 ;
ii) mesurer le signal produit par le ou les agents de détection ; et
iii) Détecter ou déterminer la présence ou la quantité de prégabaline.

**5.** Procédé selon la revendication 4, dans lequel l'anticorps est adsorbé sur ou attaché à un dispositif à l'état solide.

**6.** Procédé selon la revendication 5, dans lequel le dispositif à l'état solide est une biopuce.

**7.** Procédé selon la revendication 6, dans lequel le dispositif à l'état solide est une plaque de microtitrage.

**8.** Kit pour détecter ou déterminer la prégabaline dans un échantillon, ledit kit comprenant un anticorps selon les revendications 2 ou 3.

**9.** Kit selon la revendication 8, dans lequel l'anticorps est adsorbé sur ou attaché à un dispositif à l'état solide.

**10.** Kit selon la revendication 9, dans lequel le dispositif à l'état solide est une plaque de microtitrage ou une biopuce.

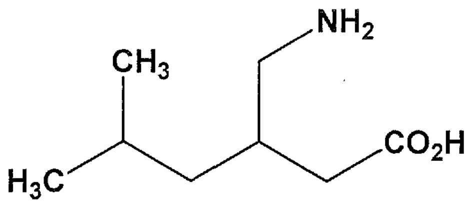

**Pregabalin**
**3-(aminomethyl)-5-methylhexanoic acid**

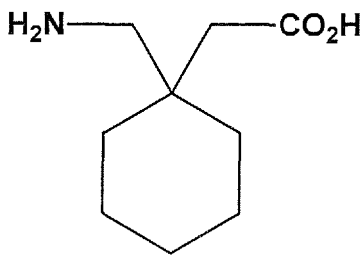

**Gabapentin**

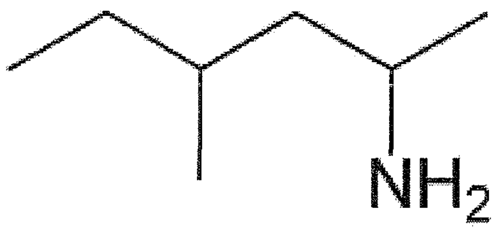

DMAA

Figure 1

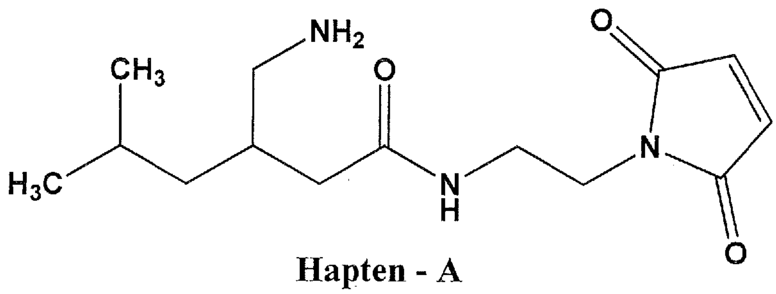

Hapten - A

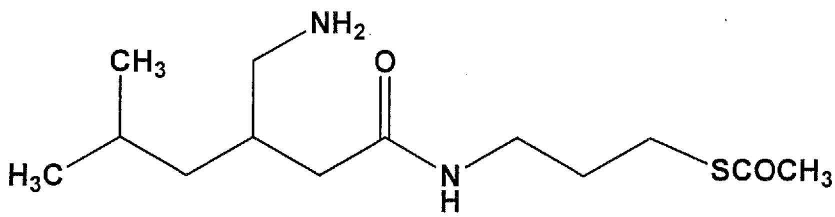

Hapten - B

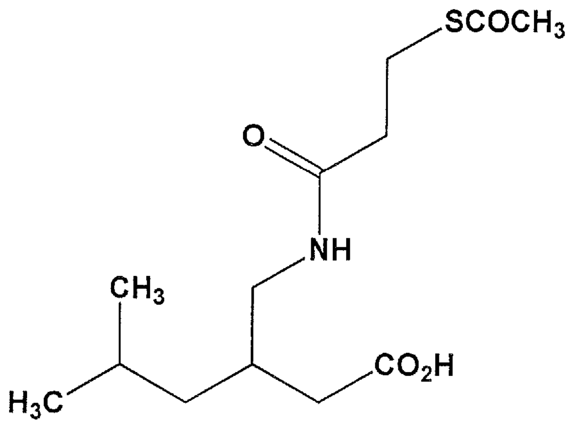

Hapten - C

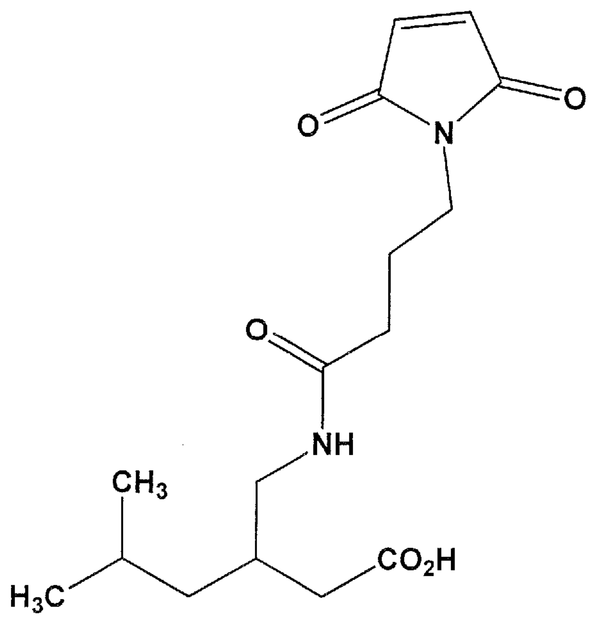

Hapten - D

Figure 2

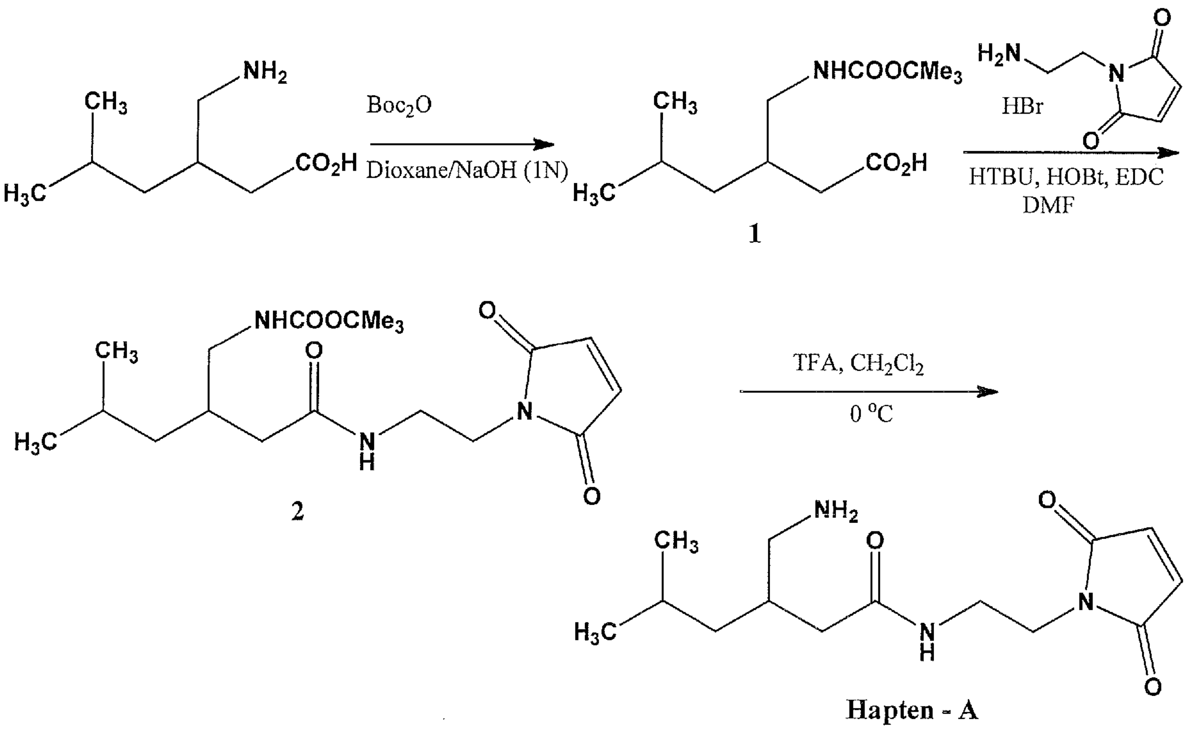


1

2

**Hapten - A**

Figure 3

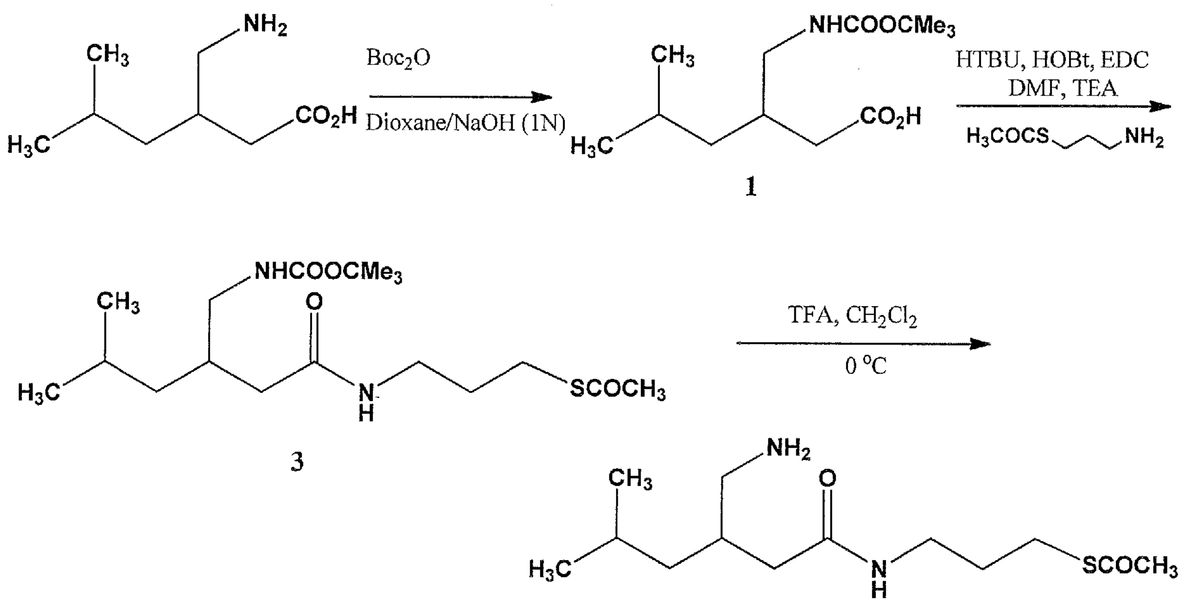


1

3

Hapten - B

Figure 4

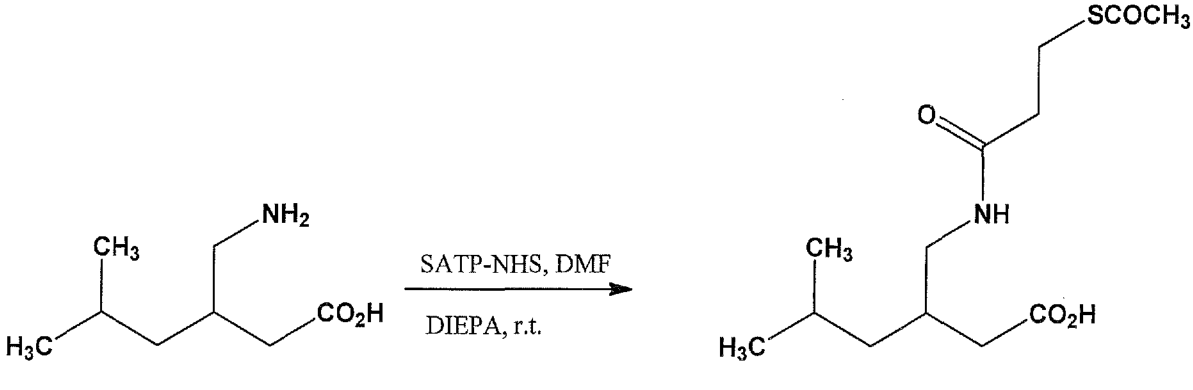


Hapten - C

Figure 5

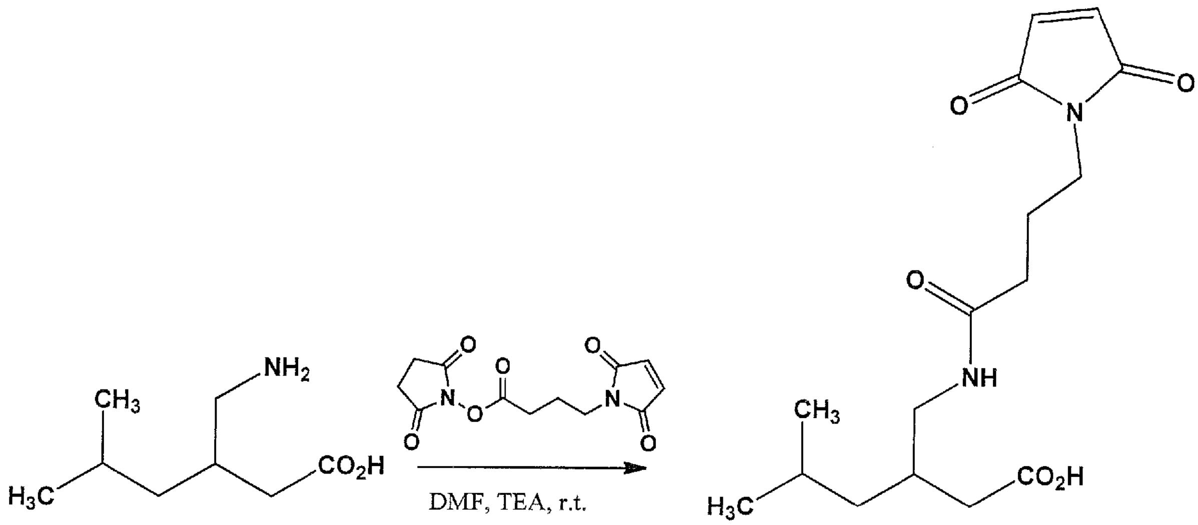


Hapten - D

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2008199887 A


### Non-patent literature cited in the description


- **AHMADKHANIHA, R et al.** *Chromatography Research International,* 2014, vol. 2014, 6
- **KRASOWSKI. M.D et al.** *Clinica Chimica Acta,* 2014, vol. 436, 224-236
- **MANDAL, U et al.** *Chromatographia,* 2008, vol. 67 (3-4), 237-243
- **SHAALAN, R. A-A.** *International Journal of Biomedical Science,* 2010, vol. 6 (3), 260-267
- **BRIAN LAW ; TAYLOR ; FRANCIS LTD.** Immunoassay: A practical guide
- **FITZGERALD, S. P. et al.** *Clin. Chem.,* 2005, vol. 51 (7), 1165-1176